# EUROPEAN PATENT SPECIFICATION

(11) **EP 4 001 288 B1**
(45) Date of publication and mention of the grant of the patent: **07.05.2025**
(21) Application number: 20208607.0
(22) Date of filing: 19.11.2020
(51) Int. Cl.: C07J 1/00, A61K 31/565, A61P 5/26, A61P 21/06

(54) **PROCESS FOR THE PREPARATION OF TRENBOLONE ACETATE HAVING A DEFINITE PARTICLE SIZE DISTRIBUTION AND A IRREGULAR HEXAGON PLATES CRYSTAL HABIT**
VERFAHREN ZUR HERSTELLUNG VON TENBOLONACETAT MIT EINER BESTIMMTEN PARTIKELGRÖSSENVERTEILUNG UND EINEM KRISTALLHABITUS MIT UNREGELMÄSSIGEN SECHSECKPLATTEN
PROCÉDÉ DE PRÉPARATION D'ACÉTATE DE TRENBOLONE PRÉSENTANT UNE DISTRIBUTION GRANULOMÉTRIQUE DÉFINIE ET UN HABITUS CRISTALLIN À PLAQUES IRRÉGULIÈRES HEXAGONALES

(43) Date of publication of application: 25.05.2022
(73) Proprietor: F.I.S.- Fabbrica Italiana Sintetici S.p.A., 36075 Montecchio Maggiore (VI) (IT)
(72) Inventor: Rencurosi, Anna, 36075 Montecchio Maggiore (VI) (IT); Brescello, Roberto, 36075 Montecchio Maggiore (VI) (IT)
(74) Representative: Leganza, Alessandro

(56) References cited:
- CN-A- 102 399 253
- CN-A- 102 924 553
- CN-A- 108 017 682
- DD-A1- 134 769
- GB-A- 1 035 683
- ZHANG HUYUE ET AL: "Synthesis of Trenbolone acetate in high yield", ACTA ACADEMIAE MEDICINAE SHANGHAI, SHANGHAI, CN, vol. 29, no. 3, 1 January 2002 (2002-01-01), pages 211 - 212, XP008084590, ISSN: 0257-8131

## Description

### Technical Field

The present invention refers to an improved process for the preparation of Trenbolone Acetate. Moreover, it is also related to a solid form of Trenbolone Acetate.

### Background Art

Trenbolone Acetate is a small molecule, sold under brand names such as Finajet and Finaplix among others, is an androgen and anabolic steroid (AAS) medication which is used in veterinary medicine, specifically to increase the profitability of livestock by promoting muscle growth in cattle.

The drug is a synthetic androgen and anabolic steroid and hence is an agonist of the androgen receptor (AR), the biological target of androgens like testosterone and dihydrotestosterone (DHT). It has strong anabolic effects and highly androgenic effects, as well as potent progestogenic effects, and weak glucocorticoid effects. Trenbolone acetate is an androgen ester and a long-lasting prodrug of trenbolone in the body.

Trenbolone acetate was discovered in 1963 and was introduced for veterinary use in the early 1970s.

Trenbolone acetate, or trenbolone 17β-acetate, is a synthetic estrane steroid and a derivative of nandrolone (19-nortestosterone). It is the C17β acetate ester of trenbolone, which itself is δ9,11-19-nortestosterone (δ9,11-19-NT) or estra-4,9,11-trien-17β-ol-3-one. Other trenbolone esters include enanthate, hexahydrobenzylcarbonate and undecanoate.

Trenbolone acetate has the following chemical formula (I): 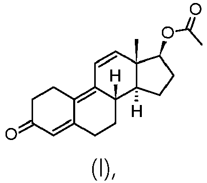 and chemical name is 17beta-acetoxy-estra-4,9,11-triene-3-one.

The described synthetic route of compound (I) is long, and some reaction yield is extremely low, so that the final product crystallization is very difficult.

Zhang et al. (in Fudan Xuebao, Yixueban 2002, 29(3), 211-212) discloses a preparation method of trenbolone acetate. The method comprises the following steps: taking estra-4,9-dien-3,17-dione (VI) as a starting raw material, carrying out protection on carbonyl group in the 3-position with methanol and p-toluenesulfonic acid as catalyst, afterwards the carbonyl group in the 17-position is reduct to hydroxy group with sodium boro hydride. The obtaining product (IV) is deprotect in acid condition to restor the carbonyl group in 3-position. The obtained (17β)-17-Hydroxyestra-5,9-dien-3-one (III) is converted tu the Trenbolone (II) by oxidation with 2,3-Dichloro-5,6-dicyano-1,4-benzoquinone (DDQ). In the last step is carry out the esterification reaction on the hydroxyl group in 17-position of Trenbolone by acetic anhydride to obtain Trenbolone acetate (I), wherein the specific route is as follows:

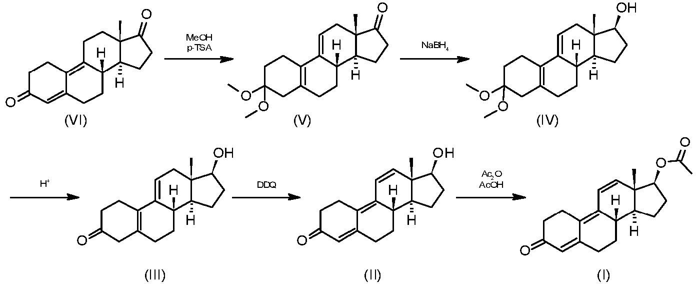

The described route has many and long steps and multiple byproducts, solid products cannot be obtained, the quality is low, the yield is low, high-toxicity solvent benzene is needed, and industrial production is not facilitated.

According to the route disclosed by the Chinese patent CN102399253, (17β)-17-Hydroxyestra-4,9-dien-3-one (VII) is used as a raw material. Diacetyl enol ester (VIII) was obtaind by acetiletation with acetyl chloride and acetic anhydride. Afterwards an hydrolysis of acetyl group in position 3 and an oxidative dehydrogenation are carried out to obtain the Trenbolone acetate, and the specific route is as follows:

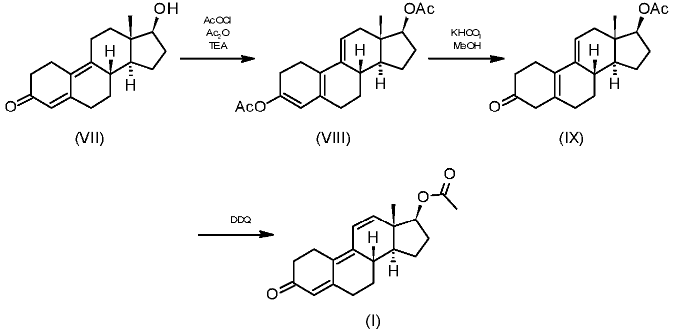

The route is firstly subjected to 17-position hydroxyl esterification, the next enol-esterification of the carbonyl group in 3 position is difficult and have a low conversion. The successive selective hydrolysis of the enol ester in 3 position is difficult, excessive hydrolysis of the acetyl group in 17-position is easy to occur. The purification of obtainied intermediate (IX) is difficult, and the scale up of process is difficult.

The route of the Chinese patent CN102924553 describe a optimized process of Zhang et al. wherein the acidic catalyst in the protection of carbonyl group in 3-position is changed from p-toluenesulfonic acid to acetyl chloride/methanol. This route uses a large amount of acidic reagent i.e. acetyl chloride, which implies large corrosion, the requirement on the production equipment is high, for which corrosion resistant equipment is required. Consequently, the waste amount is large, and the total yield is not significatilvely improved.

The route disclosed by the Chinese patent CN108017682 start from a conpound have a 4,9 open rings. Through a reduction with potassium borohydride, and a subsequent acid-catalyzed condensation, the (17β)-17-Hydroxyestra-5,9-dien-3-one (III) is obtaind. The next oxidative dehydrogenation with DDQ and the esterification, the Trenbolone acetate is obtained, and the specific route is as follows:

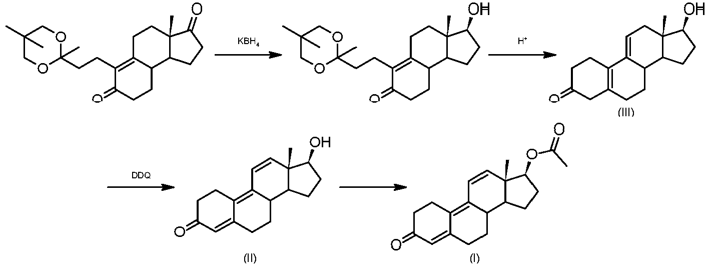

The potassium-boron hydrogen reduction is unselective and difficut to control; moreover the acid-catalyzed ring closing condensation have a low yield. Finaly, the intermediates and the product require a purification because have a low quality.

The route disclosed by the Chinese patent CN110437294 is very similar to the route discoser by Zhang et al. (in Fudan Xuebao, Yixueban 2002, 29(3), 211-212). the differences between the two ways lie in the protector group used to protect the carbonyl group in position 3, from a methanol acetal to a ethylenedioxy acetal.

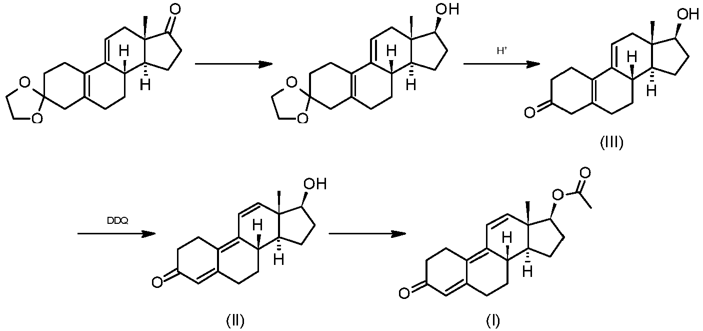

Furthermore, the disclosures CN108017682, CN102924553, CN102399253 and Fudan Xuebao, Yixueban Vol 29(3), pp 211-212 (2002) refererred to above as well as the documents GB1035683 and DD134769 all disclose the crystallisation of Trenbolone acetate from various organic solvents which do not include alcohols.

These prior art methods for the preparation of compound (I) do not describe a preparation method comprise the use of alcohol, in particular isopropanol, for the crystallization of trenbolone Acetate, moreover are silent on the PSD of the obtained product.

Gheorghe Borodi et al. in Journal of Molecular Structure 1212 (2020) 128127 describe the XPRD of Trenbolone Acetate.

These prior art methods for the preparation of Trenbolone Acetate do not allow the preparation of Trenbolone Acetate having irregular hexagon plates crystal habit, nor do they describe the crystallization by means of an alcohol, or mixture therof and do not provide a crystalline solid having irregular hexagon plates .

Furthermore, the prior art methods have the drawbacks related to the poor reproducibility of the process and of the solid form thus obtained.

Moreover, these prior art methods for the preparation of Trenbolone Acetate describe a process having a low yield and requiring a final purification step to obtain a high purity product.

Thus, there is a need for a process for preparing the intermediate Trenbolone and the final product Trenbolone Acetate in high yield and quality.

### Summary of invention

The problem addressed by the present invention is therefore that of providing a better process for crystallization of Trenbolone Acetate, which allows to get round to the drawbacks above reported with reference to the known prior art.

Moreover, the present invention is therefore that of providing a process for the preparation of Trenbolone Acetate having irregular hexagon plates crystal habit, by means of a reproducible process.

Moreover, the present invention is therefore that of providing a process for the preparation of Trenbolone Acetate having irregular hexagon plates crystal habit, by means of a reproducible process.

This problem is solved by a process for the crystallization of Trenbolone Acetate as outlined in the annexed claims, whose definitions are integral part of the present description.

Particularly, the present invention provides a process for the preparation of Trenbolone Acetate of formula (I): 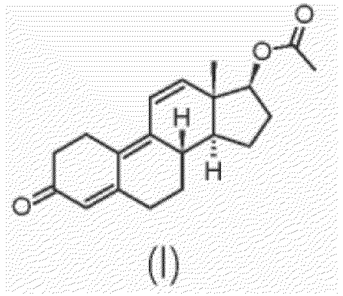 having irregular hexagon plates crystal habit, by means of crystallization of Trenbolone Acetate (I) with isopropanol.

Disclosed herein, but not forming part of the present invention is a solid form of Trenbolone Acetate, said form being Trenbolone Acetate having irregular hexagon plates crystal habit which shows the following properties:
- is thermodynamically more stable,
- and/or is in form of only irregular hexagon plates and an homogeneous solid.

Disclosed herein, but not forming part of the present invention, is Trenbolone Acetate having a specific mean particle size, which is comprised in the range between 20 and 200 microns.

Further features and advantages of the processes according to the invention will result from the description hereafter reported of examples of realization of the invention, provided as an indication of the invention.

### Drawings

Fig. 1 shows the XPRD pattern of compound of formula (I), obtained by the process of the present invention according to the examples 2 and 6.
Fig. 2 shows the DSC curve of compound of formula (I), obtained by the process of the present invention according to the examples 2 and 6.
Fig. 3 shows the SEM pattern of compound of formula (I), obtained by the process of the present invention according to the examples 2 and 6.
Fig. 4 shows DSC curve of compound (I), obtained in the example 11.
Fig. 5 shows the SEM pattern of compound (I), obtained in the example 11.
Fig. 6 shows the DSC curve of compound (I), obtained in the example 10.
Fig. 7 shows the SEM pattern of compound (I), obtained in the example 10.

### Description of embodiments

The present invention is related to a process for the preparation of Trenbolone Acetate of formula (I): 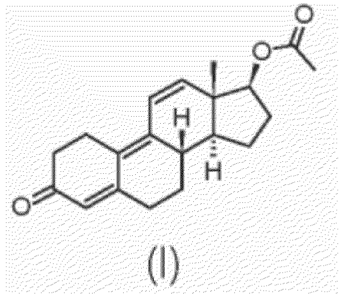 by crystallization of the Trenbolone Acetate of formula (I) with isopropanol.

In another embodiment the present invention encompasses process for preparing of Trenbolone Acetate (I), comprising the following steps:
a) providing a solution of Trenbolone Acetate in isopropanol;
b) optionally seeding said solution with a Trenbolone Acetate seed;
c) cooling until the obtainment of a suspension;
d) filtering the suspension;
e) suspending the solid obtained in the step d) in a C₅-C₁₀ alkane or in an aromatic solvent;
f) filtering the obtained suspension to obtain Trenbolone Acetate (I).

According to a preferred embodiment of the process of the present invention,the temperature during the cooling of the step c) decreases from a temperature comprised in the range from 20°C to 40°C to a temperature comprised in the range from of -20°C to 0°C.

According to a more preferred embodiment of the process of the present invention,the temperature during the cooling of the step c) decreases from a temperature comprised in the range from 20°C to 30°C to a temperature comprised in the range from of -20°C to -10°C.

According to a more preferred embodiment of the process of the present invention,the temperature during the cooling of the step c) decreases from a temperature comprised in the range from 20°C to 26°C to a temperature comprised in the range from of -18°C to -10°C.

According to a more preferred embodiment of the process of the present invention,the temperature during the cooling of the step c) decreases from a temperature comprised in the range from 20°C to 40°C to a temperature comprised in the range from of -20°C to -10°C.

According to a preferred embodiment of the process of the present invention, the temperature during the crystallization of Trenbolone Acetate of formula (I) decreases from a temperature in the range from 20°C to 40°C to the temperature of -20°C in 8 hours.

According to a preferred embodiment of the process of the present invention, the C₅-C₁₀ alkane or an aromatic solvent of the step e) is selected from the group comprising of 2-methylbutane (i.e. isopentane), cyclopentane, pentane, cyclohexane, hexane, 2-methylpentane, 3-methylpentane, 2,3-dimethylbutane, 2,2-dimethylbutane, hexane mixture of isomers, heptane, 2-methylhexane, 3-methylhexane, 2,2-dimethylpentane, 2,3-dimethylpentane, 2,4-dimethylpentane, 3,3-dimethylpentane, 3-ethylpentane, 2,2,3-trimethylbutane, methylcyclohexane, octane, methylheptane, 3-methylheptane, nonane, decane, toluene, ethylbenzene, m-xylene, o-xylene, p-xylene, mixture of xylene isomers.

According to a preferred embodiment of the process of the present invention, the C₅-C₁₀ alkane or an aromatic solvent of the step e) is selected from the group comprising of 2-methylbutane, cyclopentane, pentane, cyclohexane, hexane, hexane mixture of isomers, heptane, methylcyclohexane, octane, toluene, ethylbenzene, xylene isomer.

According to a more preferred embodiment of the process of the present invention,the C₅-C₁₀ alkane or in an aromatic solvent of the step e) is heptane.

According to a preferred embodiment of the process of the present invention, the seed of the step b) has a median value (D50) of the particle size distribution comprised in the range from 8 µm to 40 µm.

According to a preferred embodiment of the process of the present invention, the seeding of the step b) is carried out at a temperature comprised from 20°C to 30°C.

According to a more preferred embodiment of the process of the present invention, the seeding of the step b) is carried out at a temperature comprised from 20°C to 25°C.

According to a preferred embodiment of the process of the present invention, the crystallization of Trenbolone Acetate (I), with isopropanol, is seeded at a temperature comprised between 20°C and 25°C with Trenbolone Acetate of formula (I) having irregular hexagon plates crystal.

The seed of Trenbolone Acetate of formula (I) having irregular hexagon plates crystal is an amount from 0.50% w/w to 1.5% w/w referred to the entire amount of Trenbolone Acetate (I).

According to a preferred embodiment of the process of the present invention, the crystallization of Trenbolone Acetate of formula (I) is carried out in the amount of isopropanol, that is comprised between 3 and 10 volumes referred to the amount of Trenbolone Acetate (I).

The above mentioned volumes of the mixture of isopropanol and water are referred to the amount of Trenbolone Acetate (I). The amount of Trenbolone Acetate can be the weight of the starting material Trenbolone Acetate or can be determined by stoichiometric calculations assuming that all the Trenbolone of formula (II) is transformed in Trenbolone Acetate.

Volumes means volume of solvent per unit of product being the weight of Trenbolone Acetate. Thus, for example, 1 volume is 1 Liter per 1 Kilo, or 1 mL for 1 gram, or 1 microliter per 1 milligram. Thus, 10 volumes means for example 10 microliters per 1 milligram of substance, in this case, the compound of formula (I).

In the step f) Trenbolone Acetate of formula (I) is isolated, for example, by filtration or centrifugation.

The molar yield of the process according to the present invention starting from Trenbolone of formula (II) is comprised between 75% and 90%.

The molar yield of the process according to more preferred embodiment of the present invention starting from Trenbolone of formula (II) is comprised between 80% and 87%.

The molar yield of the process according to the present invention starting from Trenbolone Acetate of formula (I) is comprised between 85% and 95%.

The molar yield of the process according to the more preferred embodiment of the present invention starting from Trenbolone Acetate of formula (I) is comprised between 90% and 95%.

According to a preferred embodiment, the process of the invention provide Trenbolone Acetate (I) in high chemical purity, i.e. more than 99.50% A/A%.

In another embodiment the present invention encompasses process for preparing of Trenbolone Acetate (I), comprising the previous step of preparation of the compound (I), by acetylation of Trenbolone of formula (II):

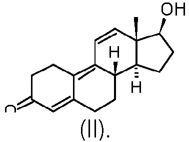

According to a preferred embodiment of the process of the present invention, the acetylation step of Trenbolone and the crystallization of the Trenbolone Acetate of formula (I) are carried out one-pot.

Specifically, the process of the invention can be carried out one-pot, i.e. starting from step of acetylation of Trenbolone of formula (II) and continuing to step of crystallization, thus producing a solid Trenbolone Acetate (I), keeping the compound of Trenbolone Acetate of formula (I) obtained in step of acetylation in a solution, i.e. without isolating it in a solid form.

In particular, at the end of the acetylation, a solvent switch to isopropanol is made thus carrying out the whole process one-pot.

Disclosed herein, but not forming part of the present invention, are pharmaceutical compositions comprising Trenbolone Acetate of formula (I): 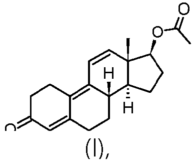 prepared according to the process of the invention and one or more pharmaceutical acceptable excipients.

According to a preferred embodiment, the process for the preparation of Trenbolone Acetate of formula (I) provides said compound of formula (I) having irregular hexagon plate crystal habit.

According to a preferred embodiment the process, the process for the preparation of Trenbolone Acetate of formula (I) provides said compound of formula (I) which in the form of only plate crystals, said solid being an homogeneous solid.

Disclosed herein, but not forming part of the present invention, is thus Trenbolone Acetate of formula (I): 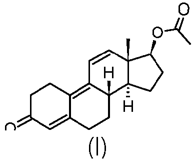 having irregular hexagon plates crystal habit wherein said Trenbolone Acetate (I) is in the form of only plates crystals.

In another embodiment the present invention encompasses process for preparing of Trenbolone Acetate of formula (I), which is has irregular hexagon plates crystal habit, said process being as defined in the claims.

Indeed the process of the present invention allows the production of Trenbolone Acetate having irregular hexagon plates crystal habit in the form of only plates crystals. It means that said solid form does not contain crystals having different morphology, but contains only plates crystals.

Trenbolone Acetate having irregular hexagon plates crystal habit in the form of only plates crystals thus does not contain any soft agglomerate or any other crystal having different morphology.

Moreover, Trenbolone Acetate having irregular hexagon plates crystal habit is an homogeneous solid.

Trenbolone Acetate having irregular hexagon plates crystal habit and which is in the form of only plate crystals can be suitably employed for the preparation of pharmaceutical compositions.

Disclosed herein, but not forming part of the present invention, is said Trenbolone Acetate (I), having irregular hexagon plate crystal habit and being in the form of only plate crystals, has mean square weight comprised in the range from 20 to 200 µm.

A mean particle size means the measure relates to mean diameter of the as determined by laser diffraction.

Also said solid form shows the same advantageous behaviour and in term of thermal stability, hygroscopicity and solubility above described.

As a further advantage of the process of the present invention, it is pointed out that the process provides an Trenbolone Acetate having irregular hexagon plate crystals with a big size and/or length, specifically, having mean square weight is comprised in the range from 20 µm to 200 µm.

The process of the invention provides Trenbolone Acetate of formula (I) having irregular hexagon plates crystal habit. In particular, the process of the present invention provides Trenbolone Acetate of formula (I) having irregular hexagon plates crystal habit which has mean square weight comprised in the range from 20 to 200 µm and/or which is in the form of only plate crystals.

The mean square weight means is determined by chord-length distribution analysis of Trenbolone Acetate of formula (I), in particular, was through by scanning electron microscopy (SEM).

The process of the present invention provides advantageously an Trenbolone Acetate having plate crystals with a big size and/or length, specifically the particle size distribution comprised in the range from 20 µm to 200 µm as determined by chord-length distribution analysis.

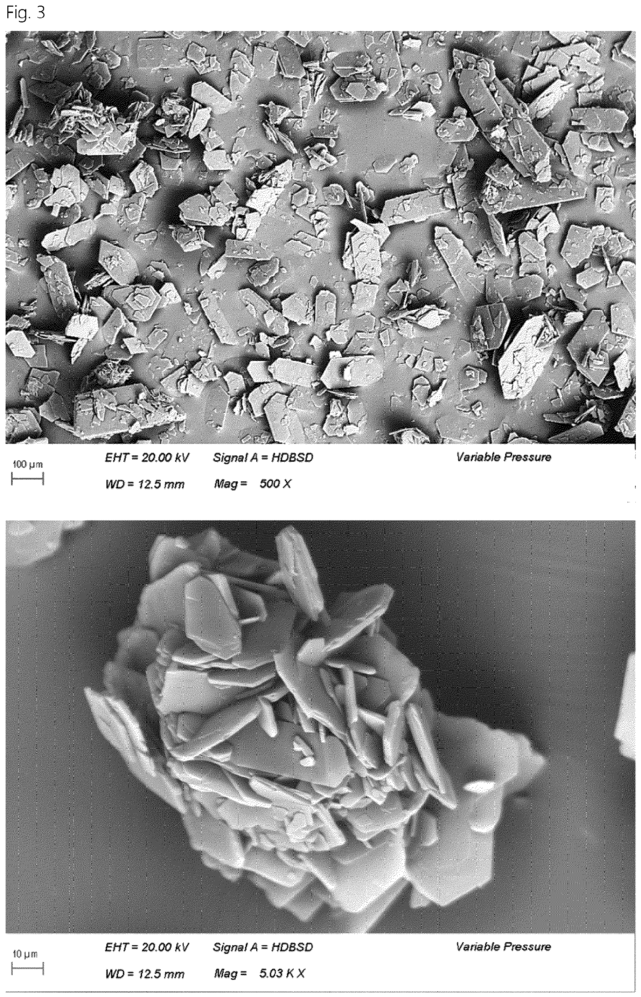

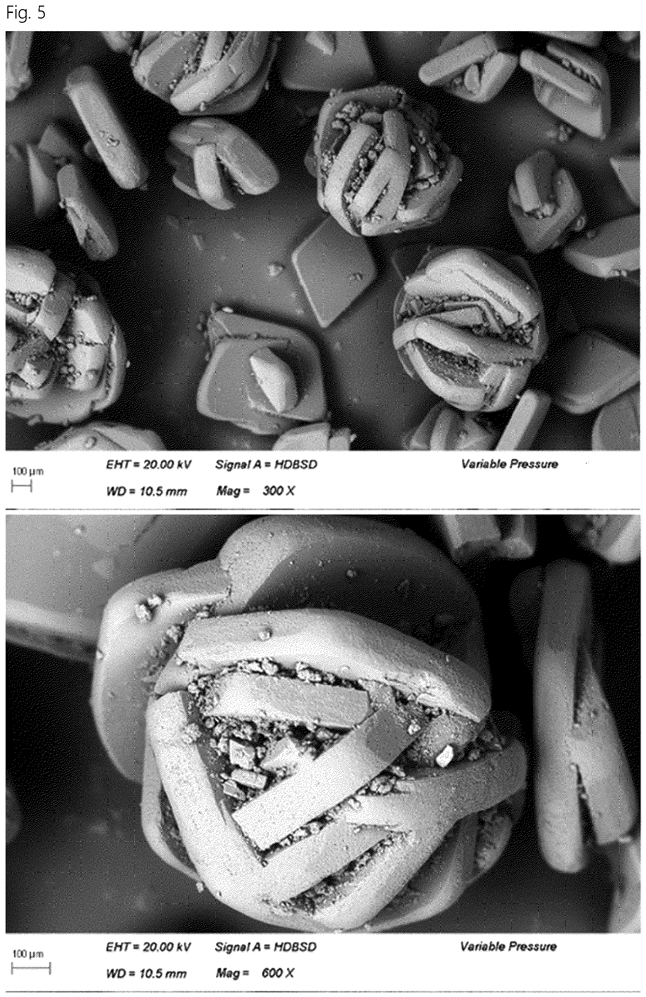

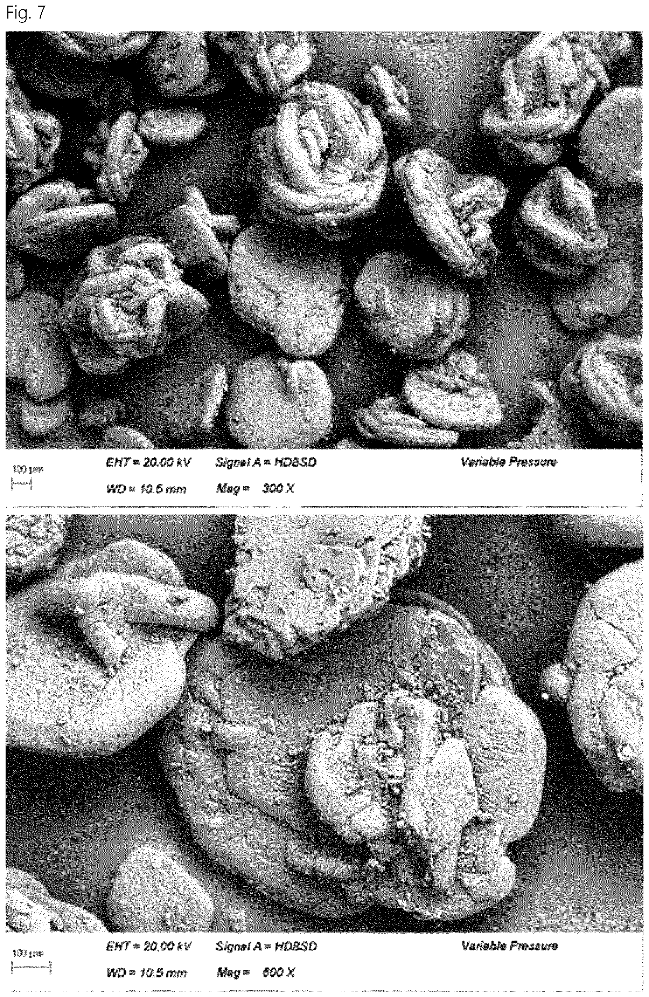

The examples 10 and 11 provides a clear comparative evidence of the effect of the present invention, indeed, Trenbolone Acetate of formula (I) prepared by means of crystallization of Trenbolone Acetate of formula (I) with a different solvent from isopropanol has not plate crystal, since it clearly has different crystal morphology (see Figure 3 versus Figures 5, 7).

The different crystal habit of the three solid forms, two obtained crystallizing with the AcOEt/Heptane mixture and with diisopropyl ether (DIPE) (non-plate crystals) and the other with isopropanol (plate crystals) was indeed well evident by microscopy analysis.

In particular, the comparison of pictures in Figure 3 and Figures 5 and 7 acquired by microscopy analysis provides a better further evidence of the different crystal habit of the three forms.

Moreover, Fig. 3 clearly show the plates crystal habit of Trenbolone Acetate obtained by means of crystallization of Trenbolone Acetate of formula (I) with isopropanol.

Moreover, Trenbolone Acetate of formula (I) having irregular hexagon plates crystal habit this feature allows the product to be arranged in a more compact way and therefore have a higher solid density, from which it allows to have more compound per volume unit; i.e. with Trenbolone Acetate of formula (I) having irregular hexagon plates crystal habit is possible to have more active ingredient for the same volume of pharmaceutical composition.

Another surprising advantage of the process of the invention is given, contrary to what is observed in the processes of the prior art, by the ease of controlling the precipitation and also by the non-aggregation of the crystallized product (i.e. no lumps are observed in the product)

Said solid form shows the same advantageous behaviour in term of stability.

Described herein, but not forming part of the present invention, is Trenbolone Acetate having irregular hexagon plates crystal habit and having a mean square weight comprised in the range from 20 to 200 µm is that in it is the form of only plate crystals. Said solid form has the advantage of being an homogeneous solid and therefore is well suitable, especially in terms of processability, for preparing pharmaceutical compositions comprising Trenbolone Acetate.

The process of the present invention provides Trenbolone Acetate of formula (I) having irregular hexagon plate crystal habit. Said Trenbolone Acetate (I) is also described by the following parameters.

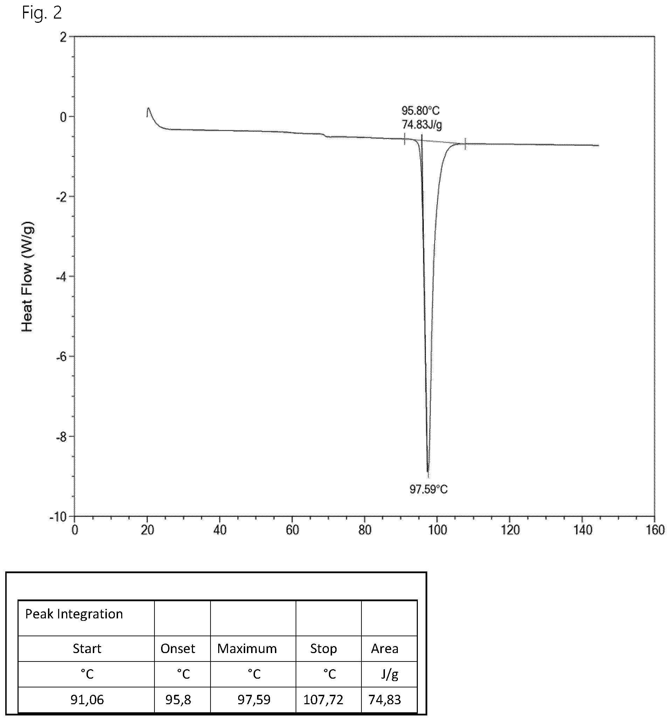

The solid form of Trenbolone Acetate having irregular hexagon plates crystal habit, obtained by means of crystallization of said Trenbolone Acetate with isopropanol, has a melting point of 95.8°C as measured by DSC (onset) (See Fig. 2).

Specifically, the value of the DSC onset and DSC peak is recorded as measured by DSC.

The DSC onset corresponds to the melting point recorded by DSC analysis, whose method is better described in the experimental part.

Moreover, Trenbolone Acetate having irregular hexagon plates crystal habit shows a peak at 97.59°C as measured by DSC.

Thus, Trenbolone Acetate having irregular hexagon plates crystal habit has a melting point of 95.8°C which is higher of that of Trenbolone Acetate having non-irregular hexagon plates crystal habit, being around 93°C, both measured by DSC (onset) (See respectively Fig. 2 and Fig. 4, 6).

Such a different thermal behavior is also confirmed by the different peak in the DSC analysis: Trenbolone Acetate having irregular hexagon plates crystal habit shows a peak at 97.59°C, an higher temperature compared with the peak at around 95°C of Trenbolone Acetate having non-irregular hexagon plates crystal habit.

The above said DSC comparative study gives thus evidence that the crystalline habit of Trenbolone Acetate being irregular hexagon plates crystals is thermodynamically more stable of the crystal habit being non-irregular hexagon plates crystals, since the melting point of the first is higher than that of the latter (97,59°C versus around 95°C (peak) and 95,8°C versus around 93°C (onset)).

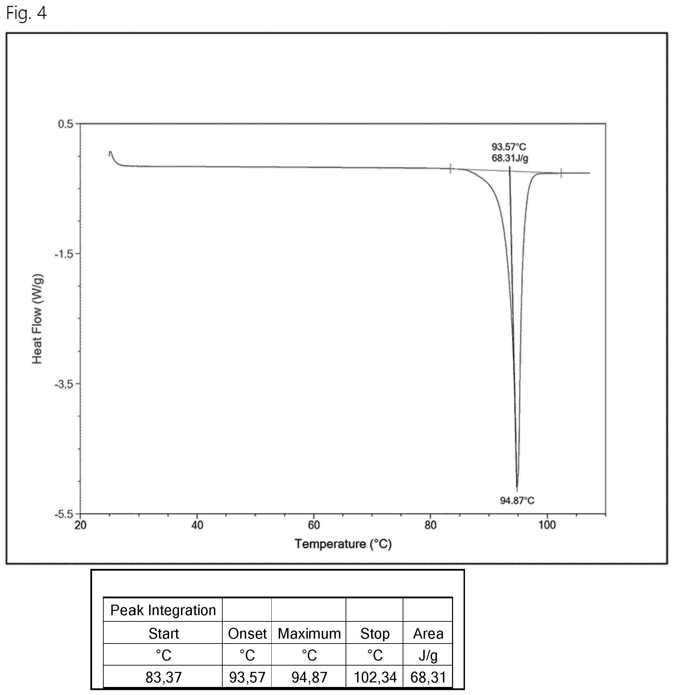

The comparison of the DSC curves of Fig. 4, 6 and Fig. 2 provides a clear evidence of the different thermal behavior of the three solid forms of Trenbolone Acetate having three different crystal habits, respectively non-plates and plate crystals, maybe of the same polymorphic form.

In particular, Trembolone Acetate having irregular hexagon plates crystal habit has a characteristic X-ray powder diffraction pattern with characteristic peaks expressed in 2-Theta values (2θ) at: 6.5, 13.2, 20.9 each peak ± 0.2 .

More in particular, Trenbolone Acetate having irregular hexagon plates crystal habit has a characteristic X-ray powder diffraction pattern with characteristic peaks expressed in 2-Theta values (2θ) at: 6.5, 13.2, 16.1, 19.5, 20.9, 22.3 each peak ± 0.2.

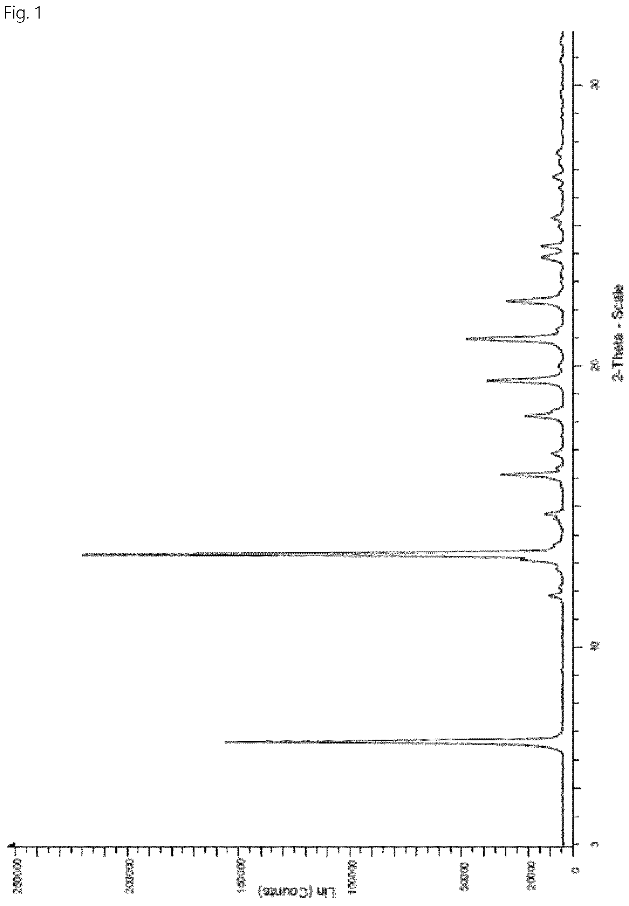

Again more particularly, Trenbolone Acetate having irregular hexagon plates crystal habit has a characteristic X-ray powder diffraction pattern with characteristic peaks expressed in 2-Theta values (2θ) at: 6.5, 13.2, 16.1, 18.2, 19.5, 20.9, 22.3, 23.8 and 24.2, each peak ± 0.2 (See Fig. 1).

In the case of Trenbolone Acetate having irregular hexagon plates crystal habit, this difference of intensity comes from the orientation preferential of the crystals caused by the plates shape.

It has been indeed surprisingly found that the crystallization of Trenbolone Acetate of formula (I), with isopropanol is a well reproducible and solid process for obtaining Trenbolone Acetate a median value (D50) of the particle size distribution comprised in the range from 50 µm to 130 µm and a irregular hexagon plates crystal habit.

It has been indeed surprisingly found that the crystallization of Trenbolone Acetate of formula (I), with isopropanol, is wel reproducible and solid process for obtaining Trenbolone Acetate a median value (D50) of the particle size distribution comprised in the range from 50 µm to 130 µm and a irregular hexagon plates crystal habit.

Described herein, but not forming part of the present invention are pharmaceutical compositions comprising Trenbolone Acetate of formula (I): 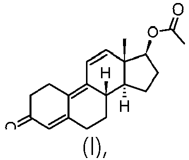 in form irregular hexagon plates crystal habit and one or more pharmaceutical acceptable excipients

Examples of suitable pharmaceutical compositions comprising Trenbolone Acetate are those disclosed in WC 2001043748A2, WO 9930685 A1, WO 9947073 A1, WO 2000025743 A2, WO 2001043749 A2, WO 2019217203 A1 or WO 2020061550 A1 with the difference that Trenbolone Acetate having irregular hexagon plates crystal habit is used instead of the disclosed Trenbolone Acetate having non-irregular hexagon plates crystal habit.

Pharmaceutical compositions have different dosage forms, which may include, for example, capsules, tablets, powders, suspensions or any other suitable dosage form. In such said dosage forms, the Trenbolone Acetate (I) having irregular hexagon plates crystal habit may be combined with one or more pharmaceutically acceptable excipients, carriers or diluents, such as, for example mannitol, silicic derivatives or sugar.

Trenbolone Acetate of formula (I) having irregular hexagon plates crystal habit, obtained by means of crystallization of Trenbolone Acetate of formula (I) with isopropanol, as above described, can be used for the preparation of a pharmaceutical composition.

Trenbolone Acetate of formula (I) having irregular hexagon plates crystal habit, obtained by means of crystallization of Trenbolone Acetate of formula (I) with isopropanol, as above described, or the beforehand above mentioned pharmaceutical composition can be used as medicament or for use in medicine or use in veterinary.

Trenbolone Acetate of formula (I) having irregular hexagon plates crystal habit, obtained by means of crystallization of Trenbolone Acetate of formula (I) with isopropanol, as above described, or the beforehand above mentioned pharmaceutical composition can be used as promoter androgen and/or increase muscle mass in cattle.

Trenbolone Acetate of formula (I) having irregular hexagon plates crystal habit indeed provides pharmaceutical having better bioavailability. According to a preferred embodiment, Trenbolone Acetate has mean particle size comprised in the range between 20 and 200 µm.

Described herein, but not forming part of the present invention, is Trenbolone Acetate having a mean particle size comprised in the range between 20 and 200 microns, has a irregular hexagon plates crystal habit. In such a case, especially, where the crystal habit does not facilitates the manufacturing process of the pharmaceutical product, the small particle size distribution provides a very positive effect, thus balancing the two effects, and providing a product which is the more stable solid form of Trenbolone Acetate.

Described herein, but not forming part of the present invention, is Trenbolone Acetate, having a mean particle size comprised in the range between 20 and 200 microns, is in the form of only plate crystals. Such a product is particularly homogenous and therefore appears to be the easiest product to be used in the manufacturing process for preparing the pharmaceutical product. Furthermore it allows a better control of the amount of the active substance dosed during process for preparing the pharmaceutical product.

Pharmaceutical composition comprising Trenbolone Acetate of formula (I) heaving irregular hexagon plates crystal habit, and one or more pharmaceutical acceptable excipients can be prepared.

Said pharmaceutical compositions, which contains Trenbolone Acetate of formula (I) heaving irregular hexagon plates crystal habit can have the same administration route and dosage forms as beforehand described related to pharmaceutical composition comprising Trenbolone Acetate having non-irregular hexagon plates crystal habit.

Described herein, but not forming part of the present invention, is the preparation of said pharmaceutical compositions, the process the following steps:
a) providing the said Trenbolone Acetate of the invention;
b) mixing the Trenbolone Acetate of step a) with a one or more pharmaceutical acceptable excipients;
c) packaging the obtained mixture of step b).

Trenbolone Acetate having plate crystalline habit, being the thermodynamically more stable solid form, exhibits therefore better storage stability and can be more easily formulated into pharmaceutical compositions of medicaments.

The process of the present invention is carried out by means of a crystallization of Trenbolone Acetate of formula (I). The crystallization, in the present case, is a process of formation of solid crystals from a solution wherein Trenbolone Acetate of formula (I) has been previously prepared and/or solubilized. In particular the term crystallization of the process of the present invention means either crystallization or recrystallization.

The recrystallization is a technique used for purifying chemical compounds and/or for obtaining a different solid forms (e.g. polymorphs), in particular a solid compound is solubilized in an appropriate solvent, and then the compound re-become a solid, typically a solid crystal, by means of heating and then cooling treatment or by solubilisation in a proper solvent and addition of an anti-solvent.

The process of the present invention can be thus, for example, carried out starting from Trenbolone Acetate and recrystallizing it by heating/cooling treatment or, alternatively, can be carried out by obtaining Trenbolone Acetate in solution and then crystallizing the product, for example, by cooling or by addition of an anti-solvent, or by other ways.

Moreover, said improved process for the preparation of Trenbolone Acetate having irregular hexagon plates crystal allows the preparation of Trenbolone Acetate having high chemical purity, i.e. more than 99.90% by HPLC A/A%.

The skilled in the art of organic chemistry can appreciate as the process of the invention allows an improvement of the productivity considering the reductions of number of steps employed to carry out the synthesis of the Trenbolone Acetate (i.e. the process does not require a recrystallization step of Trenbolone Acetate).

### EXPERIMENTAL SECTION

The starting material Trenbolone and Trenbolone Acetate can be prepared according to well-known prior art methods, or for example, as described in the CN 108017682, CN 102399253, CN 102924553 or in CN 110437294 or can be purchased on the market.

Suspension means a solid material suspended in a solvent or solution, i.e. mixture of a solid with a solvent, which is liquid. The solvent also contains other compound or a solid.

Room temperature (RT) means a temperature that is comprised in a range from 20 to 25°C, it is defined as comfortable temperature range indoors.

Molar equivalent means that the molar amount of a substance reacts with a molar amount of another substance in a given chemical reaction.

The term "volume" means volume of solvent per unit of product, thus, for example, 1 volume is 1 L per 1 Kg, or 1 mL per 1 gram, or 1 microliter per 1 milligram.

Of the following examples, example 1 is a preparatory example (showing how Trenbolone is prepared), claims 2 and 6 are examples according to the present invention and all other examples are for the purposes of comparison.

### Example 1: Preparation of Trenbolone, starting from 9(10)-Dehydronandrolone (DHN).

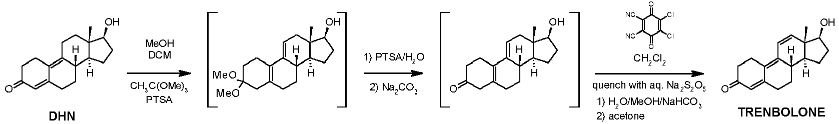

To a solution of 100 g DHN in 250 mL DCM, 50 mL MeOH and 1.4 g of PTSA is added dropwise over the course of at least 2h to a stirred solution of 100 mL DCM, 350 mL MeOH and 61 mL trimethyl orthoacetate. At the end of the addition, the addition funnel is rinsed with 33 mL DCM and the rinse transferred into the reaction mixture. After IPC analysis showing reaction completion, to the mixture was added 500 mL of water and left stirring for 0.5-2 h. The aqueous phase is discarded and to the organic phase is added a mixture of 400 mL water and 5 g PTSA. After IPC analysis showing reaction completion, the biphasic mixture is separated, and the organic phase is neutralized by addition under stirring at r.t. of a solution of water (100 mL) and NaHCO₃ (0.2 g), which brings the pH at 7-8. The phases are separated, and to the organic phase are added 200 mL DCM. The obtained solution is concentrated under vacuum to around 200 mL by keeping the internal temperature below 30 °C. This addition / concentration protocol is repeated until the solution is anhydrous. After reaching this limit, the mixture is evaporated one last time until the total volume reaches 300 mL. The mixture is treated with 24 mL of acetic acid and added dropwise to a stirred suspension of 96 g DDQ in 700 mL DCM, maintaining the internal temperature between 0-7 °C. After the end of the addition, the addition vessel is rinsed with 50 mL DCM and the rinse is combined with the reaction mixture, which is left stirring at 0-7 °C until reaching complete conversion. The reaction mixture is quenched by addition at 0-7°C of a solution composed of 38 g of aqueous Na₂S₂O₅ (30% by weight), 73 mL water and 20 mL MeOH. The obtained slurry is warmed to r.t., left stirring for 0.5 h after which it is filtered, and the filter cake is washed with 2x100 mL DCM. The obtained biphasic mixture is separated, and the organic phase washed with a solution of 200 mL water, 20 mL MeOH and 7.7 g of NaHCO₃. After phase separation, the organic layer is washed again with a solution of 200 mL water, 60 mL MeOH and 7.7 g of NaHCO₃. The phases are separated, and the organic layer washed one final time with a solution of 200 mL water, 100mL MeOH and 7.7 g NaHCO₃. The combined, filtered solution is concentrated with stirring to 300 mL under vacuum, keeping the internal temperature below 30 °C. To the resulting solution, 300 mL of acetone are added, and the obtained mixture concentrated under vacuum to 300 mL (total volume) again, keeping the internal temperature below 30 °C. The resulting suspension is cooled to 0 °C and kept at this temperature for 0.5 h with stirring, after which it is filtered, and the filter cake is washed twice with 100 mL acetone. The wet solid is dried at 40 °C max under vacuum to give Trenbolone in 65% yield and >99.0 % A/A purity by HPLC.

### Example 2: Preparation of Trenbolone Acetate, starting from Trenbolone, , by acetylation following of crystallization from Isopropanol (IPA).

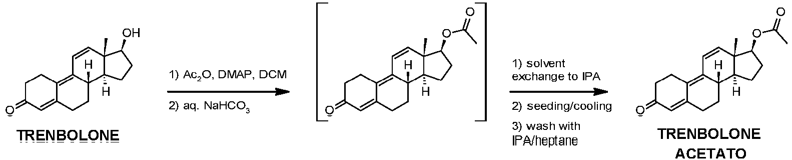

To a stirred mixture of 100 g Trenbolone and 2 g 4-DMAP in 250 mL DCM are added 60 g of acetic anhydride. The anhydride addition funnel is rinsed with 50 mL of DCM and the obtained solution is warmed to 30°C with stirring. At completed reaction, the solution is cooled to 20°C, and added to a suspension of 31 g NaHCO₃ in 300 mL water with stirring. The acetylation reactor is rinsed with additional 50 mL DCM and the rinse is added to the biphasic reaction mixture, which is then warmed again to 30°C with stirring for 1h. Then the mixture is cooled to 20°C, and the aqueous layer is discarded. The organic layer is washed with 100 mL water, and phase are separed. To the obtained organic solution is added isopropanol (300 mL) and the mixture is concentrated under vacuum to 500 mL, with the internal temperature kept at 30°C. To the mixture is added isopropanol (300 mL) again, and the solvent distilled under vacuum to 580 mL (total volume) by keeping the internal temperature at 30°C. This addition/concentration protocol is repeated three times. The solution is seeded with 0.5 g Trenbolone acetate at an internal temperature of 20-25°C. The mixture is left at 20-25°C for 30', after which it is cooled slowly to an internal temperature of -20/-10 °C. The obtained suspension is kept at -20/-10°C for 0.5 h and then filtered. The filter cake is washed with 100 mL of cold IPA, and then the resulting wet solid is triturated by resuspension in cold n-heptane (150 mL) for 0.5 h, after which the mixture is filtered and dried at 40 °C under vacuum to give Trenbolone acetate in 83% yield and >99.0% A/A by HPLC, with a PSD within D50 67-130 µm.

### Example 3: Preparation of Trenbolone Acetate, starting from Trenbolone, , by acetylation following of crystallization from Ethanol.

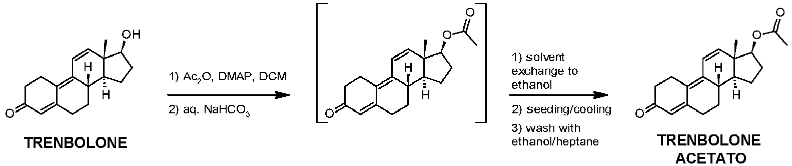

To a stirred mixture of 100 g Trenbolone and 2.2 g 4-DMAP in 250 mL DCM are added 60 g of acetic anhydride. The anhydride addition funnel is rinsed with 50 mL of DCM and the obtained solution is warmed to 30°C with stirring. At complited reaction, the solution is cooled to 20°C, and added to a suspension of 31.1 g NaHCO₃ in 300 mL water with stirring. The acetylation reactor is rinsed with additional 50 mL DCM and the rinse is added to the biphasic reaction mixture, which is then warmed again to 30°C with stirring for 1 h. Then the mixture is cooled to 20°C, and the aqueous layer is discarded. The organic layer is washed with 100 mL water, and phase are separed. To the obtained organic solution is added ethanol (300 mL) and the mixture is concentrated under vacuum to 500 mL (total volume), with the internal temperature kept at 30°C. To the mixture is added ethanol (300 mL) again, and the solvent distilled under vacuum to 580 mL (total volume) by keeping the internal temperature at 30°C. This addition/concentration protocol is repeated three times. The solution is seeded with 0.8 g Trenbolone acetate at an internal temperature of 20-25°C. The mixture is left at 20-25°C for 30', after which it is cooled slowly to an internal temperature of -20/-10 °C. The obtained suspension is kept at -20/-10°C for 0.5 h and then filtered. The filter cake is washed with 100 mL of cold ethanol, and then the resulting wet solid is triturated by resuspension in cold n-heptane (150 mL) for 0.5 h, after which the mixture is filtered and dried at 40 °C under vacuum to give Trenbolone acetate in 70% yield and >99.0% A/A by HPLC.

### Example 4: Preparation of Trenbolone Acetate, starting from Trenbolone, , by acetylation following of crystallization from n-Butanol.

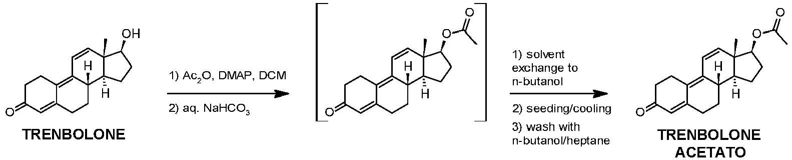

To a stirred mixture of 100 g Trenbolone and 2.2 g 4-DMAP in 250 mL DCM are added 60 g of acetic anhydride. The anhydride addition funnel is rinsed with 50 mL of DCM and the obtained solution is warmed to 30°C with stirring. At complited reaction, the solution is cooled to 20°C, and added to a suspension of 31.1 g NaHCO₃ in 300 mL water with stirring. The acetylation reactor is rinsed with additional 50 mL DCM and the rinse is added to the biphasic reaction mixture, which is then warmed again to 30°C with stirring for 1 h. Then the mixture is cooled to 20°C, and the aqueous layer is discarded. The organic layer is washed with 100 mL water, and phase are separed. To the obtained organic solution is added n-butanol (300 mL) and the mixture is concentrated under vacuum to 500 mL (total volume), with the internal temperature kept at 30°C. To the mixture is added n-butanol (300 mL) again, and the solvent distilled under vacuum to 580 mL (total volume) by keeping the internal temperature at 30°C. This addition/concentration protocol is repeated three times. The solution is seeded with 1,5 g Trenbolone acetate at an internal temperature of 20-25°C. The mixture is left at 20-25°C for 30', after which it is cooled slowly to an internal temperature of -20/-10 °C. The obtained suspension is kept at -20/-10°C for 0.5 h and then filtered. The filter cake is washed with 100 mL of cold n-butanol, and then the resulting wet solid is triturated by resuspension in cold n-heptane (150 mL) for 0.5 h, after which the mixture is filtered and dried at 40 °C under vacuum to give Trenbolone acetate in 75% yield and >99.0% A/A by HPLC.

### Example 5: Preparation of Trenbolone Acetate, starting from Trenbolone, , by acetylation following of crystallization from n-Propanol.

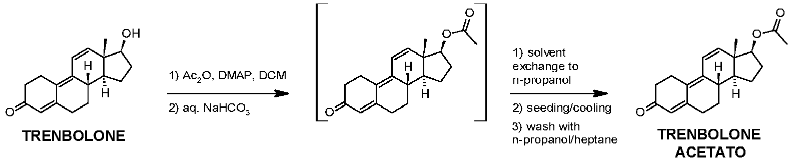

To a stirred mixture of 100 g Trenbolone and 2.2 g 4-DMAP in 250 mL DCM are added 60 g of acetic anhydride. The anhydride addition funnel is rinsed with 50 mL of DCM and the obtained solution is warmed to 30°C with stirring. At complited reaction, the solution is cooled to 20°C, and added to a suspension of 31.1 g NaHCO₃ in 300 mL water with stirring. The acetylation reactor is rinsed with additional 50 mL DCM and the rinse is added to the biphasic reaction mixture, which is then warmed again to 30°C with stirring for 1 h. Then the mixture is cooled to 20°C, and the aqueous layer is discarded. The organic layer is washed with 100 mL water, and phase are separed. To the obtained organic solution is added n-propanol (300 mL) and the mixture is concentrated under vacuum to 500 mL (total volume), with the internal temperature kept at 30°C. To the mixture is added n-propanol (300 mL) again, and the solvent distilled under vacuum to 580 mL (total volume) by keeping the internal temperature at 30°C. This addition/concentration protocol is repeated three times. The solution is seeded with 1 g Trenbolone acetate at an internal temperature of 20-25°C. The mixture is left at 20-25°C for 30', after which it is cooled slowly to an internal temperature of -20/-10 °C. The obtained suspension is kept at -20/-10°C for 0.5 h and then filtered. The filter cake is washed with 100 mL of cold n-propanol, and then the resulting wet solid is triturated by resuspension in cold n-heptane (150 mL) for 0.5 h, after which the mixture is filtered and dried at 40 °C under vacuum to give Trenbolone acetate in 78% yield and >99.0% A/A by HPLC.

### Example 6: Crystallization of Trenbolone Acetate from IPA.

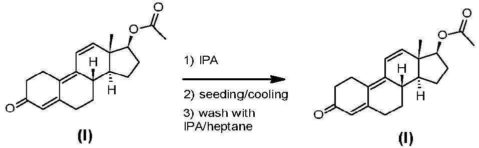

To a suspension of 50 g Trenbolone acetate in 200 mL IPA at 20°C is added methylene chloride (2.5 mL). The mixture is heated to 30°C upon which complete dissolution is observed. The solution is cooled to 20-25°C, and it is seeded with 0.5% w/w of Trenbolone acetate. The mixture is left at 20-25°C for 30', after which it is cooled slowly to an internal temperature of -15°C. The obtained suspension is kept at -15 °C for 0.5 h and then filtered. The filter cake is washed with 50 mL of cold Ethanol, and then the resulting wet solid is triturated by resuspension in cold n-heptane (75 mL) for 0.5 h, after which the mixture is filtered and dried at 40°C max under vacuum to give Trenbolone acetate in 90% yield and >99.0% A/A by HPLC.

### Example 7: Crystallization of Trenbolone Acetate from Ethanol.

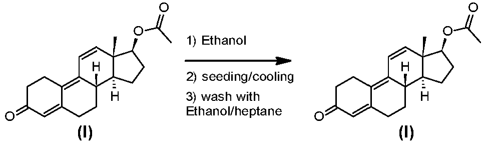

To a suspension of 50 g Trenbolone acetate in 200 mL Ethanol at 20°C is added methylene chloride (2.5 mL). The mixture is heated to 30°C upon which complete dissolution is observed. The solution is cooled to 20-25°C, and it is seeded with 0.8% w/w of Trenbolone acetate. The mixture is left at 20-25°C for 30', after which it is cooled slowly to an internal temperature of -15°C. The obtained suspension is kept at -15 °C for 0.5 h and then filtered. The filter cake is washed with 50 mL of cold Ethanol, and then the resulting wet solid is triturated by resuspension in cold n-heptane (75 mL) for 0.5 h, after which the mixture is filtered and dried at 40°C max under vacuum to give Trenbolone acetate in 70% yield and >99.0% A/A by HPLC.

### Example 8: Crystallization of Trenbolone Acetate from n-Propanol.

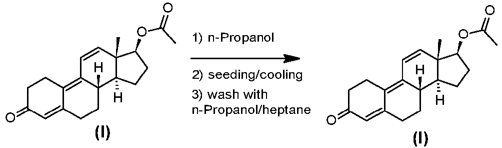

To a suspension of 50 g Trenbolone acetate in 200 mL n-Propanol at 120°C is added methylene chloride (2.5 mL). The mixture is heated to 30°C upon which complete dissolution is observed. The solution is cooled to 20-25°C, and it is seeded with 0.7% w/w of Trenbolone acetate. The mixture is left at 20-25°C for 30', after which it is cooled slowly to an internal temperature of -15°C. The obtained suspension is kept at -15 °C for 0.5 h and then filtered. The filter cake is washed with 50 mL of cold n-Propanol, and then the resulting wet solid is triturated by resuspension in cold n-heptane (75 mL) for 0.5 h, after which the mixture is filtered and dried at 40°C max under vacuum to give Trenbolone acetate in 75% yield and >99.0% A/A by HPLC.

### Example 9: Crystallization of Trenbolone Acetate from n-Butanol.

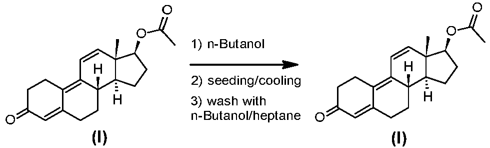

To a suspension of 50 g Trenbolone acetate in 200 mL n-Butanol at 120°C is added methylene chloride (2.5 mL). The mixture is heated to 30°C upon which complete dissolution is observed. The solution is cooled to 20-25°C, and it is seeded with 1.3% w/w of Trenbolone acetate. The mixture is left at 20-25°C for 30', after which it is cooled slowly to an internal temperature of -15°C. The obtained suspension is kept at -15 °C for 0.5 h and then filtered. The filter cake is washed with 50 mL of cold n-Butanol, and then the resulting wet solid is triturated by resuspension in cold n-heptane (75 mL) for 0.5 h, after which the mixture is filtered and dried at 40°C max under vacuum to give Trenbolone acetate in 80% yield and >99.0% A/A by HPLC.

### Example 10: Preparation of Trenbolone Acetate, by acetylation following of crystallization from diisopropyl ether (DIPE).

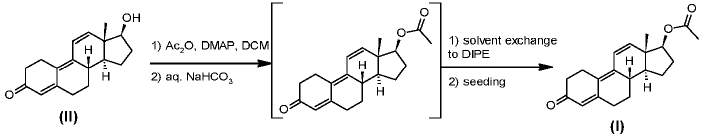

To a stirred mixture of 25 g Trenbolone and 0.3 g 4-DMAP in 187.5 mL DCM are added a total of 30 g of acetic anhydride and the obtained solution is stirred at 20°C. At complited reaction, the solution is treated with a solution of 31.1 g NaHCO₃ in 250 mL water with stirring. The aqueous layer is discarded, and the organic layer is washed with 125 mL water. After phase separation, the pooled aqueous washes are re-extracted with 125 mL DCM. The organic phases are combined and concentrated under vacuum by keeping the internal temperature below 30 °C. To the obtained solution is added diisopropyl ether (125 mL) and the mixture is concentrated under vacuum to 100 mL (total volume), with the internal temperature kept at 30°C. To the mixture is added diisopropyl ether (100 mL) again, and the solvent distilled under vacuum to 100 mL (total volume) by keeping the internal temperature at 30°C. This addition/concentration protocol is repeated three times. The mixture is cooled to -15°C and cold diisopropyl ether is added dropwise (100 mL). The solution is seeded with 0.5 g renbolone acetate at an internal temperature of -15°C. The mixture is left at -15°C for 8h, after which it is filtered. The filter cake is washed with 25 mL of cold diisopropyl ether and dried at 40°C under vacuum to give Trenbolone acetate in 69% yield and >99.0% A/A by HPLC. See Fig. 7 for the morphology.

### Example 11: Preparation of Trenbolone Acetate, by recrystallization from EtOAc/Heptane.

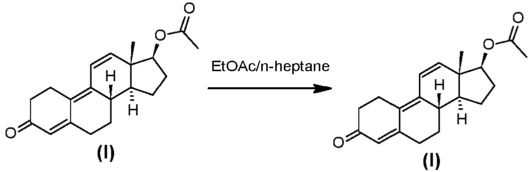

A stirred suspension of 50 g Trenbolone acetate in n-heptane (75 mL) is heated to 60 °C. Under stirring, EtOAc is added (50 mL) while maintain the internal temperature at 60 °C. To the resulting solution is added n-heptane dropwise (500 mL), while keeping the internal temperature at 60 °C. After stirring for 0.5 h at this temperature, the mixture is cooled to -10 °C in 3h. Spontaneous crystallization occurs at 40-42 °C. After reaching -10 °C, the suspension is kept at this temperature for 15 minutes, then filtered and washed with 2x25 mL portions of n-heptane (at -10 °C). The filter cake is dried at 40 °C max under vacuum to give Trenbolone acetate in 83% yield and >99.0% A/A by HPLC. See Fig. 5 for the morphology.

### Example 12: Analytical method to identify and quantify of compound of formula (I), moreover for determining the chemical purity, via HPLC:

- Colum: Hypersil-ODS, 150 × 4.0 mm, 3.0 µm, or equivalent;
- Temp. Column: 25°C;
- Mobile Phase A: Acetonitrile/Methanol/MilliQ water 36.5 : 30: 33.5 v/v/v;
- Mobile Phase B: Acetonitrile/Methanol 90 : 10 v/v;
- Gradient

| Time (min) | %A | %B |
|---|---|---|
| 0 | 100 | 0 |
| 6 | 100 | 0 |
| 16 | 0 | 100 |
| 26 | 0 | 100 |
| 26.1 | 100 | 0 |
| 30 | 100 | 0 |

- Flow: 1.0 mL/min;
- UV Detector: 229 nm;
- Injection Volume: 5 µL;
- Analysis Time: 26 min;
- Diluent: ACN/MeOH/MilliQ water/Acetic acid 36.5:30:33.5:0.1 v/v/v/v.

### Example 13: Characterization of solid form of Trenbolone Acetate having irregular hexagon plates crystal habit, prepared according to examples 2, 6.

Crystal habit: The solid form of Trenbolone Acetate has irregular hexagon plates crystal habit. Moreover, Trenbolone Acetate shows a crystalline habit as soft polycrystalline agglomerates.

DSC: method for the characterization the products of the present invention.

DSC analysis was recorded with a Mettler DSC822e. A sample of compound was charged into a 40 µL aluminium crucible with a pinhole lid and was heated, under nitrogen (50 mL/min), at 10 °C/min from 30 to 300 °C.

DSC testing was performed in a sealed medium pressure stainless steel crucible. All the testing was performed by heating the sample from 30°C at a rate of 10°C/minute up to a maximum temperature of 300°C.

DSC analysis of the crystalline compound of formula (I) obtained in the previous examples (i.e. as in examples 2, 6, 10, 11), was analysed.

The solid form of Trenbolone Acetate, obtained according to the inventio, is characterized by an endothermic peak corresponding to the melting point with an onset at 97,59°C (fusion enthalpy -74,83 J/g), measured by DSC analysis. At 95,8°C (onset) begin a endotermic peak (as shown in Figure 2).

The solid form of Trenbolone Acetate, obtained according to the example 11, is characterized by an endothermic peak corresponding to the melting point with an onset at 94,87°C (fusion enthalpy 68,31 J/g), measured by DSC analysis. At 93,57°C (onset) begin a broad endotermic peak (see Fig. 4).

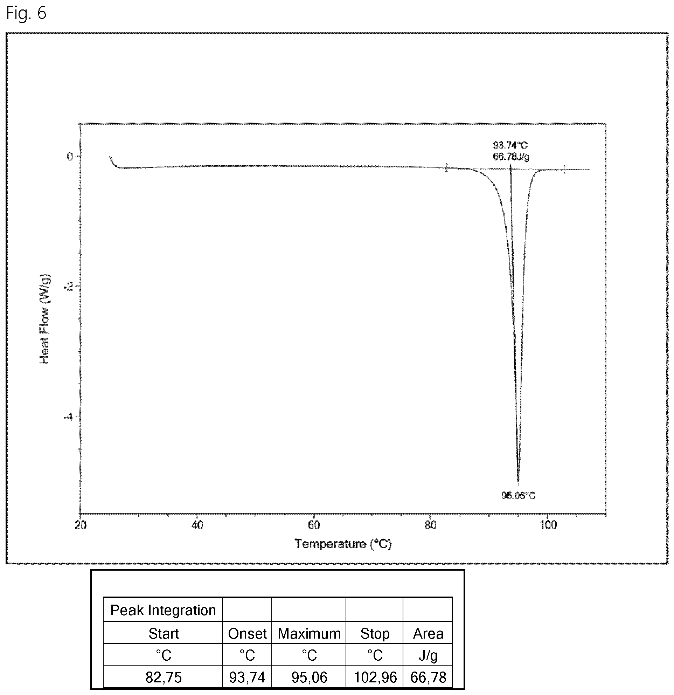

The solid form of Trenbolone Acetate, obtained according to the example 10, is characterized by an endothermic peak corresponding to the melting point with an onset at 95,06°C (fusion enthalpy -66,78 J/g), measured by DSC analysis. At 93,74°C (onset) begin a broad endotermic peak (see Fig. 6).

Microscopy analysis: the solid of Trenbolone Acetate (I) was analysed by microscopy for identification of crystal habits.

Microscope was recorded with a Zeiss Evo MA10 scanning electron microscope. A sample of powder has been gold spattered and observed by SEM at different magnification, micrographs have been collected at same conditions and magnification (20 kV, HDBSD, Variable Pressure). All the samples have been observed after same treatment and same time after preparation.

Indeed, the microscopy analysis provides a better further evidence of the crystal habit of Trenbolone Acetate (I) of the examples 10 and 11 (see respective Fig.5 and 7) which has a form more different than/to Trenbolone Acetate (I) having a irregular hexagon plates crystal habit, obtained according to above described examples 2 and 6 (see Fig. 3).

Trenbolone acetate crystallized with a different solvent from a C₂-C₅ alkyl alcohol (as prepared in examples 10 and 11) presents a crystal habit forming soft polycrystalline agglomerates (see Fig. 5 and 7). There are large roundish particles constituted by packed thick plates or flakes, a number of rhomboidal isolated crystals is also detectable in both samples.

Microscopy analysis of Trenbolone Acetate having irregular hexagon plates crystal habit (as prepared in examples 2 and 9).

Trenbolone Acetate (I) having irregular hexagon plates crystal habit, crystallized with a C₂-C₅ alkyl alcohol (as prepared in examples from 2 to 9) presents a crystal habit of mainly thin irregular hexagon plates in the range 20-200 micron. There is a limited number of aggregates, constituted by tightly packed small plates while the majority are larger, shard-like particles and resemble (see Fig. 3). Individual crystals, like plates, only, in particular, thin plates.

In conclusions, microscopy analysis showed that crystals obtained with a process of present invention, presents a structure formed by big/long plates.

XRPD analysis: method for the characterization of the following products of the present invention. As far as the XRPD method is concerned, the instrument (Bruker D8 Advance diffractometer), the instrumental parameters and the other parameters used are reported in table below:

| | |
|---|---|
| Configuration | Bragg-Brentano (θ/θ) |
| Source | Cu: 40 mA, 40 kV |
| Radiations | K(α1) e K(α2) |
| Optic settings | 1. Primary Soller slit: 2.3° |
| | 2. Divergent slit: 0,3° |
| | 3. Anti-scattering slit: 3 mm |
| | 4. Secondary Soller slit: 1.5° |
| | 5. Nickel Kβ filter: 0.5 mm |
| Detector | (Lynx eye, Bruker) PSD 0,8° |
| Scan | 3.00° to 40.00° 2θ |
| Scan mode | Continuous |
| Operative conditions | 1. step size 0.015°, |
| | 2. collection time 0,5 sec per step |
| Sample holder* | 1. round in polycarbonate, 0 background, flat; |

| | |
|---|---|
| *Recommend to use a 0-background sample holder without cavity to obtain a profile with a good peak resolution | |

Trenbolone Acetate of formula (I) as prepared in examples 2, 6 were analyzed and the XRPD diffractogram are shown in Fig. 1.

Particle size distribution (PSD): In particular, particle size distribution was determined with a Malvern Mastersizer 3000, and the other parameters used are reported in table below:

| Dispersing accessory setting | |
|---|---|
| Sample handing unit | Hydro MV |
| Stirrer speed (rpm) | 2500 |
| Built-in Ultrasound power (%) | OFF |

| Material properties | |
|---|---|
| Particle refractive index | 0 |
| Absorption | 0 |
| Scattering Model | Mie |
| Dispersant name | Water 0.1%wt Tween 80 |
| Dispersant refractive index | 1.33 |
| Analysis sensitivity | Normal |
| Single result mode | No |
| Non spherical particle type | Yes |

| Measurement | |
|---|---|
| Sample measurement time (red and blue) | 20 s (12000 snaps) |
| Background measurement time (red and blue) | 20s (12000 snaps) |
| Background stability time out | 120 s |
| Use background check | Yes |
| Background check limits | [1,200], [20,20] |
| Number of measurements | 4 |
| Delay between measurement | 20 s |
| Result unit | volume |
| Obscuration limit | 12.8 -17,3% |

Particle size distribution of Trenbolone Acetate obtained according to the process of the invention shows a gaussian distribution with the D50 value was from 50 to 130 µm.

## Claims

1. Process for the preparation of Trenbolone Acetate of formula (I): 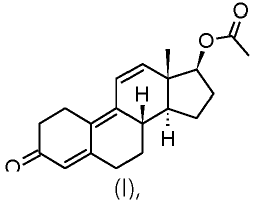
wherein Trenbolone Acetate has irregular hexagon plates crystal habit,
by crystallization of the Trenbolone Acetate of formula (I) with a C₂-C₅ alkyl alcohol, wherein the C₂-C₅ alkyl alcohol is isopropanol.

2. Process according to claim 1, comprising the following steps:
a) providing a solution of Trenbolone Acetate in isopropanol;
b) optionally seeding said solution with a Trenbolone Acetate seed;
c) cooling until the obtainment of a suspension;
d) filtering the suspension;
e) suspending the solid obtained in the step d) in a C₅-C₁₀ alkane or in an aromatic solvent;
f) filtering the obtained suspension to obtain Trenbolone Acetate of formula (I).

3. Process according to the claim 2, wherein the temperature during the cooling of the step c) decreases from a temperature comprised in the range from 20°C to 40°C to a temperature comprised in the range from of -20°C to -10°C.

4. Process according to any one of the claims from 2 to 3, wherein the C₅-C₁₀ alkane or an aromatic solvent of the step e) is heptane.

5. Process according to any one of the claims from 1 to 4, wherein Trenbolone Acetate of formula (I) has a median value (D50) of the particle size distribution comprised in the range from 50 µm to 130 µm;.

6. Process according to any one of the claims from 1 to 5, wherein the Trenbolone Acetate of formula (I) has irregular hexagon plates crystal habit.

7. Process according to any one of the claims from 1 to 6, comprising the previous step of preparation of the compound of formula (I): 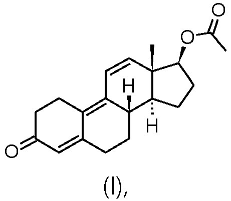 by acetylation of Trenbolone of formula (II):

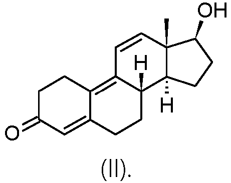

8. Process according to the claim 7, wherein the steps of the claim 2 and the previous step of claim 7 are carried out one-pot.

9. Process according to claim 1 wherein, Trenbolone Acetate has a melting point of 95.8°C as measured by DSC (onset).

## Patentansprüche

1. Verfahren zur Herstellung von Trenbolonacetat mit der Formel (I): 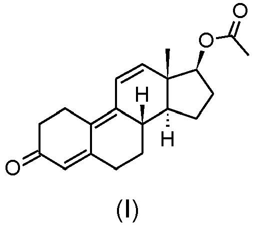
wobei Trenbolonacetat einen Kristallhabitus mit unregelmäßigen Sechseckplatten hat,
durch Kristallisation des Trenbolonacetats mit der Formel (I) mit einem C₂- bis Cs-Alkylalkohol, wobei der C₂- bis Cs-Alkylalkohol Isopropanol ist.

2. Verfahren nach Anspruch 1, das die folgenden Schritte umfasst:
a) Bereitstellen einer Lösung von Trenbolonacetat in Isopropanol;
b) gegebenenfalls Impfen der Lösung mit einem Trenbolonacetat-Impfkristall;
c) Kühlen, bis eine Suspension erhalten wird;
d) Filtrieren der Suspension;
e) Suspendieren des in Schritt d) erhaltenen Feststoffs in einem C₅- bis C₁₀-Alkan oder in einem aromatischen Lösungsmittel;
f) Filtrieren der erhaltenen Suspension, um Trenbolonacetat mit der Formel (I) zu erhalten.

3. Verfahren nach Anspruch 2, wobei die Temperatur während des Kühlens in Schritt c) von einer Temperatur, die im Bereich von 20 °C bis 40 °C liegt, auf eine Temperatur sinkt, die im Bereich von -20 °C bis -10 °C liegt.

4. Verfahren nach einem der Ansprüche 2 bis 3, wobei das C₅- bis C₁₀-Alkan oder ein aromatisches Lösungsmittel in dem Schritt e) Heptan ist.

5. Verfahren nach einem der Ansprüche 1 bis 4, wobei Trenbolonacetat mit der Formel (I) einen Medianwert (D50) der Partikelgrößenverteilung hat, der im Bereich von 50 µm bis 130 µm liegt.

6. Verfahren nach einem der Ansprüche 1 bis 5, wobei das Trenbolonacetat mit der Formel (I) einen Kristallhabitus mit unregelmäßigen Sechseckplatten hat.

7. Verfahren nach einem der Ansprüche 1 bis 6, des Weiteren, umfassend den vorhergehenden Schritt des Herstellens der Verbindung mit der Formel (I): 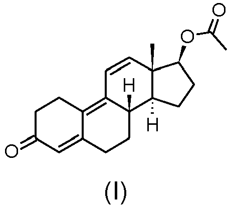 durch Acetylierung von Trenbolon mit der Formel (II):

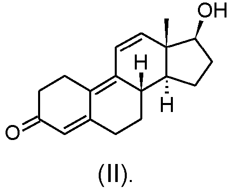

8. Verfahren nach Anspruch 7, wobei die Schritte nach Anspruch 2 und der vorhergehende Schritt nach Anspruch 7 als Eintopfreaktion durchgeführt werden.

9. Verfahren nach Anspruch 1, wobei Trenbolonacetat einen Schmelzpunkt von 95,8 °C hat, gemessen mittels DSC (Beginn des Schmelzens).

## Revendications

1. Procédé pour la préparation d'acétate de trenbolone de formule (I) : 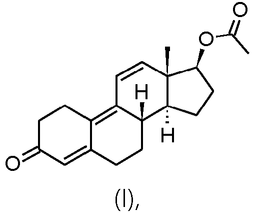
l'acétate de trenbolone présentant une forme cristalline sous forme de plaquettes hexagonales irrégulières,
par cristallisation de l'acétate de trenbolone de formule (I) avec un alcool alkylique en C₂-C₅, l'alcool alkylique en C₂-C₅ étant l'isopropanol.

2. Procédé selon la revendication 1, comprenant les étapes suivantes :
a) fourniture d'une solution d'acétate de trenbolone dans de l'isopropanol ;
b) ensemencement éventuel de ladite solution par une semence d'acétate de trenbolone ;
c) refroidissement jusqu'à l'obtention d'une suspension ;
d) filtration de la suspension;
e) mise en suspension du solide obtenu dans l'étape d) dans un alcane en C₅-C₁₀ ou dans un solvant aromatique ;
f) filtration de la suspension obtenue en vue d'obtenir l'acétate de trenbolone de formule (I).

3. Procédé selon la revendication 2, la température pendant le refroidissement de l'étape c) diminuant à partir d'une température située dans la plage de 20 °C à 40 °C jusqu'à une température située dans la plage de -20 °C à -10 °C.

4. Procédé selon l'une quelconque des revendications 2 à 3, l'alcane en C₅-C₁₀ ou un solvant aromatique de l'étape e) étant l'heptane.

5. Procédé selon l'une quelconque des revendications 1 à 4, l'acétate de trenbolone de formule (I) présentant une valeur médiane (D50) de la distribution granulométrique située dans la plage de 50 µm à 130 µm.

6. Procédé selon l'une quelconque des revendications 1 à 5, l'acétate de trenbolone de formule (I) présentant une forme cristalline sous forme de plaquettes hexagonales irrégulières.

7. Procédé selon l'une quelconque des revendications 1 à 6, comprenant l'étape préalable de préparation du composé de formule (I) : 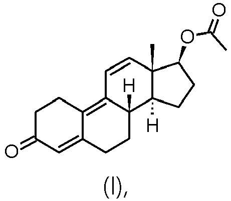 par acétylation de trenbolone de formule (II) :

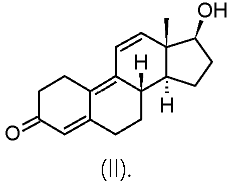

8. Procédé selon la revendication 7, les étapes de la revendication 2 et l'étape préalable de la revendication 7 étant effectuées de manière monotope.

9. Procédé selon la revendication 1, l'acétate de trenbolone présentant un point de fusion de 95,8 °C, tel que mesuré par DSC (calorimétrie différentielle à balayage) (démarrage).
